# EUROPEAN PATENT SPECIFICATION

(11) **EP 2 983 482 B1**
(45) Date of publication and mention of the grant of the patent: **20.09.2017**
(21) Application number: 14716988.2
(22) Date of filing: 24.03.2014
(51) Int. Cl.: A23C 9/12, A23L 33/16, A61K 31/19, A61K 31/7016, A61K 31/715, A61K 33/26, A61P 7/06

(54) **FERMENTED NUTRITION HIGH IN LACTOSE WITH INCREASED IRON BIOAVAILABILITY**
FERMENTIERTER NÄHRSTOFF MIT HOHEM LACTOSEGEHALT UND ERHÖHTER EISENBIOVERFÜGBARKEIT
NUTRITION FERMENTÉE RICHE EN LACTOSE AYANT UNE BIODISPONIBILITÉ DE FER ACCRUE

(30) Priority: 22.03.2013 WO PCT/NL2013/050211
(43) Date of publication of application: 17.02.2016
(73) Proprietor: N.V. Nutricia, 2712 HM Zoetermeer (NL)
(72) Inventor: VAN DEN BRAAK, Claudia Catharina Maria, 3584 CT Utrecht (NL); LUDWIG, Thomas, NL-3584 CT Utrecht (NL); BOURITIUS, Houkje, NL-3584 CT Utrecht (NL)
(74) Representative: Nederlandsch Octrooibureau
(86) International application number: PCT/NL2014/050181
(87) International publication number: WO 2014/148910

(56) References cited:
- WO-A1-2011/114916
- JP-A- H0 753 391
- US-A1- 2009 098 190
- US-A1- 2010 297 261
- DATABASE BIOSIS [Online] BIOSCIENCES INFORMATION SERVICE, PHILADELPHIA, PA, US; 2009, YEH YI-NING ET AL: "Effect of Lactose and Milk Protein on Iron Bioavailability during in vitro Digestion", XP002716453, Database accession no. PREV200900423486 & NIPPON SHOKUHIN KAGAKU KOGAKU KAISHI, vol. 56, no. 6, 2009, pages 331-335, ISSN: 1341-027X
- DATABASE EMBASE [Online] ELSEVIER SCIENCE PUBLISHERS, AMSTERDAM, NL; 1973, BOUVET D: "Study in the rat of the effect of prolonged ingestion of lactose on the iron content in ferritin and hemosiderin", XP002716518, Database accession no. EMB-0008788570 & C. R. ACAD. SCI. SER. D 1973, vol. 276, no. 2, 1973, pages 177-180,

## Description

### FIELD OF THE INVENTION

The present invention is in the field of nutritional compositions for, in particular fermented nutritional compositions, for preventing iron deficiency, in particular intended for infants and young children or pregnant women.

### BACKGROUND OF THE INVENTION

Iron plays several vital roles in the body, as it is present in hemoglobin, cytochromes in the electron transport chain, and some enzymes. Iron deficiency is one of the most common nutritional deficiencies. Iron deficiency can turn into anaemia, the most common nutritional disorder in the world, wherein the body's stores of iron have been depleted and the body is unable to maintain levels of haemoglobin in the blood. Especially infants and young children and pregnant women are prone to this disease, as they have increased iron needs, and the WHO has estimated that 43% of the world's infants and young children suffer from it. Iron deficiency has serious consequences for the health and development of infants and young children. A lack of sufficient supply or uptake of iron during the first year of life has for instance been shown to negatively impact neural development and that this negative impact can be irreversible.

Iron is taken up from the diet in the gastro-intestinal tract, in particular by absorption of the enterocytes of the duodenal lining. Iron can be absorbed as part of a protein, or in its ferrous Fe²⁺ form. Iron in its ferric, Fe³⁺, form is first converted to its ferrous form. Iron is taken up by a membrane transporter DMT1 into the enterocyte cell, where it is stored and bound to apoferritin to form ferritin or it is moved further into the body by ferroportin. The body regulates the iron levels by regulating each of these steps.

Iron in human milk is well absorbed by infants and has a high bioavailability; over 50% of the iron in human milk is absorbed as compared to less than 12% of the iron in standard infant formula.

Fermentation of bovine milk has been reported to increase iron bioavailability (Branca and Rossi 2002, Eur J Clin Nutr, 56, S16-S20). During fermentation of milk lactose is converted to lactic and short chain fatty acids concomitant with a reduction in the pH, which is speculated to increase iron absorption.

WO 2011/114916 discloses the use of Bifidobacteria as an active ingredient to add to milk to prevent or treat anaemia for pregnant/nursing mothers or infants/toddlers.

A similar disclosure is found in US 2009/0098190 and JP H07 53391.

The effect of lactose (4.8%, 6%, 7.2%) and milk protein on iron bioavailability during in vitro Digestion are discussed by Yeh Yi-Ning in Nihon-Shokuhin-Kagaku-Kogakkai-shi, 2009, VOL - 54 NR - 6 PG - 331 - 335,

Bouvet studied in C. R. Acad. Sci. Ser. D, 1973, VOL - 276 NR - 2 PG - 177 - 180 the effect of prolonged ingestion of lactose on the iron content in ferritin and hemosiderin. Sazawal et al, 2010, JPGN 51, 341-346, discloses *B. lactis* HN109 and prebiotic oligosaccharide added to milk to result in a smaller number of iron-deficient preschoolers and increased weight gain.

### SUMMARY OF THE INVENTION

The inventors, employing an *in vitro* model with Caco-2 cells and measuring intracellular ferritin concentrations as a parameter for iron bioavailability, found that iron bioavailability was increased in fermented nutritional compositions compared to non-fermented nutritional compositions. Surprisingly the presence of high amounts of lactose in the fermented nutritional compositions increased iron bioavailability to a much higher extent than the presence of high amounts of lactose increased iron bioavailability in non-fermented nutritional compositions. Unexpectedly, the presence of non-digestible oligosaccharides further increased iron uptake in the fermented nutritional compositions with high lactose, whereas no such effect of non-digestible oligosaccharides was observed in non-fermented nutritional compositions with high lactose.

Therefore the fermented nutritional compositions of the present invention comprising a high amount of lactose, and preferably non-digestible oligosaccharides, can be used to improve the iron uptake and bioavailability. Therefore the fermented nutritional compositions of the present invention comprising a high amount of lactose, and preferably non-digestible oligosaccharides, can be used to treat or prevent iron deficiency or anaemia. This is especially important for infants and young children, since these subjects have an increased need for iron and sufficient uptake of iron is important for good growth and development. Treating or preventing iron deficiency or anaemia is also especially important for pregnant women, since these subjects have an increased need for iron and sufficient uptake of iron is important for good growth and development of the unborn infant.

Therefore the fermented nutritional compositions of the present invention comprising a high amount of lactose, and preferably non-digestible oligosaccharides, also enable the formulation of nutritional compositions with a lower concentration of iron. Nutritional compositions with lower concentrations of iron beneficially have a lower competition with zinc absorption and a reduced entry of unabsorbed iron in the colon, which results in an improved colonic microbiota. Furthermore, such a nutritional composition with reduced iron has product technological advantages in that sensitive components, in particular long chain polyunsaturated fatty acids, are not peroxidised.

### DETAILED DESCRIPTION OF THE INVENTION

The present invention concerns a method for treating and/or preventing anaemia and/or treating and/or preventing iron deficiency in a human subject comprising administering to the human subject a nutritional composition comprising
a) a milk-derived product that is fermented by lactic acid producing bacteria, the fermented milk-derived product comprising lactic acid and/or lactate, and
b) at least 6 g lactose per 100 ml nutritional composition and/or at least 40 wt.% lactose based on dry weight of the nutritional composition, and
c) iron.

The invention can also be worded as the use of a milk-derived product that is fermented by lactic acid producing bacteria, the fermented milk-derived product comprising lactic acid and/or lactate in the manufacture of a nutritional composition for treating and/or preventing anaemia and/or treating and/or preventing iron deficiency in a human subject, the nutritional composition further comprising i) at least 6 g lactose per 100 ml nutritional composition and/or at least 40 wt.% lactose based on dry weight of the nutritional composition, and ii) iron.

The invention can also be worded as a nutritional composition comprising
a) a milk-derived product that is fermented by lactic acid producing bacteria, the fermented milk-derived product comprising lactic acid and/or lactate, and
b) at least 6 g lactose per 100 ml nutritional composition and/or at least 40 wt.% lactose based on dry weight of the nutritional composition, and
c) iron
for use in treating and/or preventing anaemia and/or treating and/or preventing iron deficiency in a human subject.

In one embodiment, treating and/or preventing anaemia and/or treating and/or preventing iron deficiency is in a human subject with an age of 0 to 36 months.

In one embodiment, treating and/or preventing anaemia and/or treating and/or preventing iron deficiency is in a pregnant woman.

The present invention concerns a method for increasing iron absorption, increasing iron bioaccessibility and/or increasing iron bioavailability in a human subject, comprising administering to the human subject a nutritional composition comprising
a) a milk-derived product that is fermented by lactic acid producing bacteria, the fermented milk-derived product comprising lactic acid and/or lactate, and
b) at least 6 g lactose per 100 ml nutritional composition and/or at least 40 wt.% lactose based on dry weight of the nutritional composition, and
c) iron.

In one embodiment, the method for increasing iron absorption, increasing iron bioaccessibility and/or increasing iron bioavailability in a human subject is a non-medical method.

The invention can also be worded as the use of a milk-derived product that is fermented by lactic acid producing bacteria, the fermented milk-derived product comprising lactic acid and/or lactate in the manufacture of a nutritional composition for increasing iron absorption, increasing iron bioaccessibility and/or increasing iron bioavailability in a human subject, the nutritional composition further comprising i) at least 6 g lactose per 100 ml nutritional composition and/or at least 40 wt.% lactose based on dry weight of the nutritional composition, and ii) iron.

The invention can also be worded as a nutritional composition comprising
a) a milk-derived product that is fermented by lactic acid producing bacteria, the fermented milk-derived product comprising lactic acid and/or lactate, and
b) at least 6 g lactose per 100 ml nutritional composition and/or at least 40 wt.% lactose based on dry weight of the nutritional composition, and
c) iron
for use in increasing iron absorption, increasing iron bioaccessibility and/or increasing iron bioavailability in a human subject.

In one embodiment, increasing iron absorption, increasing iron bioaccessibility and/or increasing iron bioavailability is in a human subject with an age of 0 to 36 months.

In one embodiment, increasing iron absorption, increasing iron bioaccessibility and/or increasing iron bioavailability is in a pregnant woman.

The invention also concerns a method for improving cognitive development, improving motor development and/or improving socio-emotional development in a human subject with an age of 0 to 36 months or for preventing cognitive disorders, motor disorders and/or socio-emotional disorders in a human subject with an age of 0 to 36 months, comprising administering to the human subject with an age of 0 to 36 months a nutritional composition comprising
a) a milk-derived product that is fermented by lactic acid producing bacteria, the fermented milk-derived product comprising lactic acid and/or lactate and at least one selected from the group consisting of milk, whey, whey protein, whey protein hydrolysate, casein and casein hydrolysate, and
b) at least 6 g lactose per 100 ml nutritional composition and/or at least 40 wt.% lactose based on dry weight of the nutritional composition, and
c) iron.

The invention can also be worded as the use of a milk-derived product that is fermented by lactic acid producing bacteria, the fermented milk-derived product comprising lactic acid and/or lactate in the manufacture of a nutritional composition for improving cognitive development, motor development and/or socio-emotional development in a human subject with an age of 0 to 36 months or for preventing cognitive disorders, motor disorders and/or socio-emotional disorders in a human subject with an age of 0 to 36 months, the nutritional composition further comprising i) at least 6 g lactose per 100 ml nutritional composition and/or at least 40 wt.% lactose based on dry weight of the nutritional composition, and ii) iron.

The invention can also be worded as a nutritional composition comprising
a) a milk-derived product that is fermented by lactic acid producing bacteria, the fermented milk-derived product comprising lactic acid and/or lactate, and
b) at least 6 g lactose per 100 ml nutritional composition and/or at least 40 wt.% lactose based on dry weight of the nutritional composition, and
c) iron
for use in improving cognitive development, motor development and/or socio-emotional development in a human subject with an age of 0 to 36 months or for preventing cognitive disorders, motor disorders and/or socio-emotional disorders in a human subject with an age of 0 to 36 months.

The invention also concerns a nutritional composition comprising
a) at least 25 wt.% based on dry weight of the nutritional composition of a milk-derived product that is fermented by lactic acid producing bacteria comprising lactic acid and/or lactate, and
b) at least 6 g lactose per 100 ml nutritional composition and/or at least 40 wt.% lactose based on dry weight of nutritional composition and
c) iron in a concentration of 0.4 to 0.7 mg per 100 ml nutritional composition and/or of 0.03 to 0.055 mg per g dry weight of nutritional composition.

In one embodiment, the nutritional composition that comprises at least 25 wt.% based on dry weight of the nutritional composition of a milk-derived product that is fermented by lactic acid producing bacteria comprising lactic acid and/or lactate, comprises 0.1 to 1.5 wt.% of the sum of lactic acid and lactate based on dry weight of the nutritional composition, and/or 0.01 to 0.20 g of the sum of lactic acid and/or lactate per 100 ml nutritional composition, wherein the sum of L-lactic acid and L-lactate is more than 50 wt.% based on the sum of total lactic acid and lactate.

Alternatively the invention concerns a nutritional composition comprising
a) 0.1 to 1.5 wt.% of the sum of lactic acid and lactate based on dry weight of the nutritional composition, and/or 0.01 to 0.20 g of the sum of lactic acid and/or lactate per 100 ml nutritional composition, wherein the sum of L-lactic acid and L-lactate is more than 50 wt.% based on the sum of total lactic acid and lactate, and
b) at least 6 g lactose per 100 ml nutritional composition and/or at least 40 wt.% lactose based on dry weight of nutritional composition and
c) iron in a concentration of 0.4 to 0.7 mg per 100 ml nutritional composition and/or of 0.03 to 0.055 mg per g dry weight of nutritional composition.

In one embodiment, the nutritional composition that comprises 0.1 to 1.5 wt.% of the sum of lactic acid and lactate based on dry weight of the nutritional composition, and/or 0.01 to 0.20 g of the sum of lactic acid and/or lactate per 100 ml nutritional composition, wherein the sum of L-lactic acid and L-lactate is more than 50 wt.% based on the sum of total lactic acid and lactate, comprises at least 25 wt.% based on dry weight of the nutritional composition of a milk-derived product that is fermented by lactic acid producing bacteria comprising lactic acid and/or lactate.

It is noted that wherever in the present description wording like "the present nutritional composition" or "nutritional composition according to the (present) invention" is used, this also refers to the methods and uses according to the present invention.

### Dietary iron.

The present nutritional composition comprises iron. In the context of this invention, iron means Fe²⁺ or Fe³⁺. Preferably the nutritional composition comprises non-haem iron, more preferably one or more iron sources selected from the group consisting of ferrous sulphate, ferrous lactate, ferrous gluconate, ferrous bisglycinate, ferrous citrate, ferrous fumarate, ferric diphosphate, and ferric ammonium citrate, more preferably ferrous sulphate and ferrous lactate. Wherever in this description an amount or concentration of iron is mentioned, this refers to the amount or concentration of Fe²⁺ or Fe³⁺, hence excluding the weight of the counter ion such as sulphate, lactate gluconate, etc., of the iron source. Sources of ferrous iron are preferred as sources of ferric iron need to be converted to ferrous iron in the body, the capacity of which may be limited in human subjects with an age of 0 to 36 months, e.g. infants and young children.

The present nutritional composition preferably comprises at least 0.2 mg iron per 100 ml, more preferably at least 0.4 mg per 100 ml. The present nutritional composition preferably comprises at least 0.015 mg iron per g dry weight, more preferably at least 0.03 mg per g dry weight. The present nutritional composition preferably comprises at least 0.3 mg iron per 100 kcal, more preferably at least 0.6 mg per 100 kcal. A minimal amount is preferred in order to ensure sufficient iron uptake and prevent iron deficiency.

The present nutritional compositions preferably comprises not more than 1.7 mg iron per 100 ml, more preferably not more than 1.4 mg iron per 100 ml, more preferably not more than 0.9 mg iron per 100 ml, even more preferably not more than 0.7 mg iron per 100 ml. The present nutritional compositions preferably comprises not more than 0.1 mg iron per g dry weight, more preferably not more than 0.065 mg iron per g dry weight, even more preferably not more than 0.055 mg iron per g dry weight. The present nutritional compositions preferably comprises not more than 3 mg iron per 100 kcal, more preferably not more than 2 mg iron per 100 kcal, even more preferably not more than 1.3 mg iron per 100 kcal. Too much iron can result in poor product quality by peroxidising polyunsaturated acids and can have adverse health effects. The found improved iron bioavailability of iron allows for slightly lower iron concentrations than typically present in infant formulae.

### Fermented milk-derived product

Fermentation is the process of deriving energy from the oxidation of carbohydrates, such as the lactose present in milk, using an endogenous electron acceptor, which is usually an organic compound. This is in contrast to cellular respiration, where electrons are donated to an exogenous electron acceptor, such as oxygen, via an electron transport chain. In the present invention fermentation of a milk-derived product by lactic acid producing bacteria has the common meaning of the conversion of carbohydrates present in the milk-derived product to organic acids. These organic acids formed may comprise, besides lactic acid, also other organic acids such as acetate. Lactic acid bacteria are also referred to as lactic acid producing bacteria and include bacteria of the genus Streptococcus, Lactococcus, Lactobacillus, Leuconostoc, Enterococcus, Oenococcus, Pediococcus, and Bifidobacterium. Preferably the milk-derived product is not fermented by *Lactobacillus bulgaricus. L. bulgaricus* fermented products are considered not suitable for infants, since in infants the specific dehydrogenase that converts D-lactate to pyruvate is far less active than the dehydrogenase which converts L-lactate.

According to the present invention, the nutritional composition comprises a milk-derived product that is fermented by lactic acid producing bacteria. The fermented milk-derived product comprises lactic acid and/or lactate. Preferably the fermented milk-derived product further comprises one or more selected from the group consisting of whey, whey protein, whey protein hydrolysate, casein and casein hydrolysate. The term 'fermented milk-derived product' includes fermented milk. The fermented milk-derived product is obtained by incubation of a combination of milk, e.g. skim milk, or by incubation of a combination of a composition comprising lactose and preferably one or more selected from the group consisting of whey, whey protein, whey protein hydrolysate, casein and casein hydrolysate, with at least one lactic acid producing bacterium, preferably *Streptococcus thermophilus.* Preferably the combination is incubated for 10 minutes to about 6 hours. The temperature during incubation is preferably from 20 to 50 °C. After incubation the incubated product is preferably subjected to a heat treatment. By this heat treatment preferably at least 90 % of living lactic acid bacteria are inactivated. Thus in one embodiment according to the present invention the lactic acid producing bacteria in the nutritional composition are inactivated and/or non-replicating. The heat treatment preferably is performed at a temperature from 80 to 180 °C. Procedures to prepare fermented milk-derived products suitable for the purpose of the present invention are known per se. EP 778885, which is incorporated herein by reference, discloses in particular in example 7 a suitable process for preparing a fermented milk-derived product. FR 2723960, which is incorporated herein by reference, discloses in particular in example 6 a suitable process for preparing a fermented milk-derived product.

Briefly, a milk-derived product, preferably pasteurised, containing lactose and optionally further macronutrients such as (vegetable) fats, casein, whey protein, vitamins and/or minerals etc. is concentrated, e.g. to a value of 15 to 50% dry matter and then inoculated with *S. thermophilus*, for example with 5% of a culture containing 10⁶ to 10¹⁰ bacteria per ml. Temperature and duration of fermentation are as mentioned above. Suitably after fermentation the fermented milk-derived product may be pasteurised or sterilised and for example spray dried or lyophilised to provide a form suitable to be formulated in the end product.

The bacterial strains of *S. thermophilus* that are preferably used to prepare the fermented milk-derived product for the purpose of the present develop beta-galactosidase activity in the course of fermentation of the substrate. Preferably beta-galactosidase activity develops in parallel with acidity. Preferably a beta-galactosidase activity develops which is sufficient to permit subsequent enrichment of the fermented milk-derived product in galactooligosaccharides. Thus preferably suitable *S. thermophilus* strains, when cultured on a medium containing lactose, in particular a medium based on milk concentrate, achieve fermentation of the medium accompanied by high production of galactooligosaccharides. Selection of a suitable strain of *S. thermophilus* is described in example 2 of EP 778885 and in example 1 of FR 2723960. A preferred strain of *S. thermophilus* is then selected that with a developing beta-galactosidase activity also produce galactooligosaccharides. Preferred strains of *S. thermophilus* to prepare the fermented milk-derived products for the purpose of the present invention have been deposited by Compagnie Gervais Danone at the Collection Nationale de Cultures de Microorganismes (CNCM) run by the Institut Pasteur, 25 rue du Docteur Roux, Paris, France on 23 August 1995 under the accession number I-1620 and on 25 August 1994 under the accession number I-1470. Preferably, in the preparation of the fermented milk-derived product additionally other strains of lactic acid bacteria are present or, either simultaneously or consecutively, the fermented milk-derived product additionally is fermented by other strains of lactic acid bacteria. Other strains of lactic acid bacteria are preferably selected from the group consisting of Lactobacillus and Bifidobacteria, more preferably *Bifidobacterium breve,* most preferably Bifidobacterium *B. breve* strain deposited by Compagnie Gervais Danone at the CNCM under number I-2219 on 31 May, 1999. Preferably the milk-derived product is fermented by *Streptococcus thermophilus* and/or *Bifidobacterium breve.* Preferably the nutritional composition comprises *Streptococcus thermophilus* and/or *Bifidobacterium breve.* Preferably the lactic acid producing bacteria in the nutritional composition are present in inactivated and/or non-replicating form, as it prevents the further degradation of the high amounts of lactose present in the product.

The present composition preferably comprises at least 5 wt.% based on dry weight of the total product, of the fermented milk-derived product. Preferably the composition comprises at least 10 wt.%, more preferably at least 25 wt.%, even more preferably at least 40 wt.% based on dry weight of the total product, of the fermented milk-derived product. The present composition comprises at most 100 wt.% based on dry weight of the total product, of the fermented milk-derived product. Preferably the composition comprises at most 90 wt.%, more preferably at most 70 wt.%, even more preferably at most 50 wt.% based on dry weight of the total product, of the fermented milk-derived product.

The present nutritional composition comprises lactic acid and/or lactate. Lactic acid and/or lactate is formed upon fermentation by lactic acid producing bacteria. Preferably the present nutritional composition comprises from 0.1 to 1.5 wt.% lactic acid and/or lactate, more preferably from 0.2 to 1.0 wt.%, based on dry weight of the nutritional composition. In one embodiment, preferably the present nutritional composition comprises from 0.01 to 0.20 g lactic acid and/or lactate per 100 ml of nutritional composition. More preferably the present nutritional composition comprises from 0.03 to 0.14 g lactic acid and/or lactate per 100 ml of nutritional composition. It is noted that the amounts given relate to the sum of lactic acid and lactate in case both are present. Preferably at least 50 wt. %, even more preferably at least 90 wt.%, of the sum of lactic acid and lactate is in the form of L-isomer. Thus in one embodiment the sum of L-lactic acid and L-lactate is more than 50 wt. %, more preferably more than 90 wt.%, based on the sum of total lactic acid and lactate. L-lactate and L-lactic acid is the same as L-(+)-lactate and L-(+)-lactic acid. It is noted that one of the sources of iron can be ferrous lactate. In case ferrous lactate is selected as source of iron, the amount of lactate therefrom is in addition to the amount of lactate that is formed upon fermentation by lactic acid producing bacteria. Thus, in one embodiment, in case the source of iron is ferrous lactate, the present nutritional composition preferably comprises from 0.1 to 1.6 wt.% lactic acid and/or lactate, more preferably from 0.2 to 1.1 wt.%, based on dry weight of the nutritional composition. Also in one embodiment, in case the source of iron is ferrous lactate, preferably the present nutritional composition comprises from 0.01 to 0.21 g lactic acid and/or lactate per 100 ml of nutritional composition. More preferably the present nutritional composition comprises from 0.03 to 0.15 g lactic acid and/or lactate per 100 ml of nutritional composition. The common isomer of lactate in ferrous lactate is L-(+)-lactate.

### Lactose

The present nutritional composition comprises lactose, preferably the present nutritional composition comprises at least 6 g lactose per 100 ml, more preferably at least 6.5 g, even more preferably at least 7 g lactose per 100 ml. The present nutritional composition comprises preferably at least 40 wt.% lactose based on dry weight of the nutritional composition, more preferably at least 45 wt.%, even more preferably at least 50 wt.% based on dry weight of the nutritional composition. Preferably the present nutritional composition comprises at least 8.5 g lactose per 100 kcal, more preferable at least 9.5 g, even more preferable at least 10 g per 100 kcal. Preferably the nutritional composition comprises at least 60 wt.%, more preferably at least 70 wt.% even more preferably at least 90 wt.% lactose based on total digestible carbohydrate.

The present nutritional composition comprises preferably no more than 20 g lactose per 100 ml, more preferably no more than 15 g, even more preferably no more than 10 g lactose per 100 ml. The present nutritional composition comprises preferably no more than 90 wt.% lactose based on dry weight of the nutritional composition, more preferably no more than 80 wt.%, even more preferably no more than 70 wt.% based on dry weight of the nutritional composition. Preferably the present nutritional composition comprises no more than 30 g lactose per 100 kcal, more preferably no more than 20 g, even more preferably no more than 15 g per 100 kcal.

The high amount of lactose in a fermented product surprisingly resulted in an improved iron bioavailability. The combination of a fermented product and a high concentration of lactose is uncommon, as the lactose is typically converted upon fermentation into organic acids. In order to prevent this further fermentation of lactose in the product, the product is for example in dry form such as a powder. Another way is by inactivating the lactic acid producing bacteria and/or the lactase enzyme activity, for example by a heat treatment. Another way is by inactivating the lactic acid producing bacteria and/or the lactase enzyme activity, for example by storage at low temperature. Preferably the product is in dry form, more preferably in powder form.

### Non-digestible oligosaccharides

The present nutritional composition preferably comprises non-digestible oligosaccharides. Non-digestible oligosaccharides were found to further enhance iron bioavailability in a nutritional composition with fermented milk-derived product and high concentration of lactose. Advantageously and most preferred, the non-digestible oligosaccharides are water-soluble (according to the method disclosed in L. Prosky et al, J. Assoc. Anal. Chem 71: 1017-1023, 1988) and are preferably oligosaccharides with a degree of polymerisation (DP) of 2 to 200. The average DP of the non-digestible oligosaccharides are preferably below 200, more preferably below 100, even more preferably below 60, most preferably below 40. The non-digestible oligosaccharides are not digested in the intestine by the action of digestive enzymes present in the human upper digestive tract (small intestine and stomach). The non-digestible oligosaccharides are fermented by the human intestinal microbiota. For example, glucose, fructose, galactose, sucrose, lactose, maltose and the maltodextrins are considered digestible. The oligosaccharide raw materials may comprise monosaccharides such as glucose, fructose, fucose, galactose, rhamnose, xylose, glucuronic acid, GalNac etc., but these are not part of the oligosaccharides as in the present invention. The non-digestible oligosaccharides included in the nutritional compositions and methods according to the present invention preferably include a mixture of non-digestible oligosaccharides.

The non-digestible oligosaccharides are preferably selected from the group consisting of fructooligosaccharides, such as inulin, non-digestible dextrins, galactooligosaccharides, such as transgalactooligosaccharides, xylooligosaccharides, arabinooligosaccharides, arabinogalactooligosaccharides, glucooligosaccharides, gentiooligosaccharides, glucomannooligosaccharides, galactomannooligosaccharides, mannanoligosaccharides, isomaltooligosaccharides, nigerooligosaccharides, glucomannooligosaccharides, chitooligosaccharides, soy oligosaccharides, uronic acid oligosaccharides, sialyloligosaccharides, such as 3-sialyllactose (3-SL), 6-sialyllactose (6-SL), lactosialylterasaccharide (LST) a,b,c, disialyllactoNtetraose (DSLNT), sialyl-lactoNhexaose (S-LNH), DS-LNH, and fucooligosaccharides, such as (un)sulphated fucoidan oligosaccharides, 2'-fucosyllactose (2'-FL), 3-FL, difucosyllactose, lacto-N-fucopenatose, (LNFP) I, II, III, V, Lacto-N-neofucopenaose (LNnFP), Lacto-N-difucosyl-hexaose (LNDH), and mixtures thereof, even more preferably selected from the group consisting of fructooligosaccharide, such as inulin, galactooligosaccharide, such as transgalactooligosaccharide, uronic acid oligosaccharide and fuco-oligosaccharide and mixtures thereof, even more preferably transgalactooligosaccharide, inulin and/or uronic acid oligosaccharides, most preferably transgalactooligosaccharides. In one embodiment in the composition or methods according to the present invention, the non-digestible oligosaccharides are selected from the group consisting of transgalactooligosaccharides, fructooligosaccharides and galacturonic acid oligosaccharides and mixtures of thereof.

The non-digestible oligosaccharides are preferably selected from the group consisting of β-galactooligosaccharide, α-galactooligosaccharide, and galactan. According to a more preferred embodiment non-digestible oligosaccharides are β-galactooligosaccharide. Preferably the non-digestible oligosaccharides comprises galactooligosaccharides with β(1,4), β(1,3) and/or β(1,6) glycosidic bonds and a terminal glucose. Transgalactooligosaccharide is for example available under the trade name VIVINAL^{®}GOS (Borculo Domo Ingredients, Zwolle, Netherlands), Bi2muno (Clasado), Cup-oligo (Nissin Sugar) and Oligomate55 (Yakult).

The non-digestible oligosaccharides preferably comprise fructooligosaccharides. A fructooligosaccharide may in other contexts have names like fructopolysaccharides, oligofructose, polyfructose, polyfructan, inulin, levan and fructan and may refer to oligosaccharides comprising β-linked fructose units, which are preferably linked by β(2,1) and/or β(2,6) glycosidic linkages, and a preferable DP from 2 to 200. Preferably, the fructooligosaccharide contains a terminal β(2,1) glycosidic linked glucose. Preferably, the fructooligosaccharide contains at least 7 β-linked fructose units. In a further preferred embodiment inulin is used. Inulin is a type of fructooligosaccharide wherein at least 75% of the glycosidic linkages are β(2,1) linkages. Typically, inulin has an average chain length from 8 to 60 monosaccharide units. A suitable fructooligosaccharide for use in the compositions of the present invention is commercially available under the trade name RAFTILINE^{®}HP (Orafti). Other suitable sources are RAFTILOSE^{®} (Orafti), FIBRULOSE^{®} and FIBRULINE^{®} (Cosucra) and FRUTAFIT^{®}t and FRUTALOSE^{®} (Sensus).

In one embodiment, the non-digestible oligosaccharides comprise a mixture of galactooligosaccharides and fructooligosaccharides. Preferably the mixture of galactooligosaccharides and fructooligosaccharides is present in a weight ratio of from 1/99 to 99/1, more preferably from 1/19 to 19/1, even more preferably from 1 to 19/1. This weight ratio is particularly advantageous when the galactooligosaccharides have a low DP and the fructooligosaccharides have a relatively high DP. Preferably the non-digestible oligosaccharides comprise a mixture of galactooligosaccharides with an average DP below 10, preferably below 6 and fructooligosaccharides with an average DP above 7, preferably above 11, even more preferably above 20. In one embodiment, the non-digestible oligosaccharides comprise a mixture of galactooligosaccharides and short chain fructooligosaccharides. Preferably the mixture of galactooligosaccharides and short chain fructooligosaccharides is present in a weight ratio of from 1/99 to 99/1, more preferably from 1/19 to 19/1, even more preferably from 1 to 19/1. Preferably the non-digestible oligosaccharides comprise a mixture of galactooligosaccharides with an average DP below 10, preferably below 6 and short chain fructooligosaccharides with an average DP below 10, preferably below 6.

In the embodiments above, preferably the galactooligosaccharides are transgalactooligosaccharides.

In one embodiment, the non-digestible oligosaccharides comprise a mixture of short chain fructooligosaccharides and long chain fructooligosaccharides. Preferably the mixture of short chain fructooligosaccharides and long chain fructooligosaccharides is present in a weight ratio of from 1/99 to 99/1, more preferably from 1/19 to 19/1, even more preferably from 1/2 to 19/1, or alternatively in 2/1 to 1/2, preferably about 1 to 1. Preferably the non-digestible oligosaccharides comprise a mixture of fructooligosaccharide with an average DP below 10, preferably below 6 and a fructooligosaccharide with an average DP above 7, preferably above 11, even more preferably above 20.

The present nutritional composition preferably comprises 1.0 to 20 wt.% total non-digestible oligosaccharide, more preferably 1 to 10 wt.%, even more preferably 2 to 10 wt.%, most preferably 2.0 to 7.5 wt.%, based on dry weight of the present composition.

Based on 100 ml the present nutritional composition preferably comprises 0.2 to 2.5 g total non-digestible oligosaccharide, more preferably 0.2 to 1.5 g, even more preferably 0.4 to 1.5 g, based on 100 ml of the present composition.

### Nutritional compositions

The present nutritional composition is preferably particularly suitable for providing the complete daily nutritional requirements to an infant or a young child, or in other words to a human subject with an age of 0 to 36 months, more preferably to an infant, or in other words to a human subject with an age of 0 to 12 months. The present nutritional composition is preferably not a yogurt, since yoghurt contains by convention L. bulgaricus (Codex Standard for fermented Milks Codex Stan 243-2003).

The present nutritional composition comprises digestible carbohydrate, in particular lactose. Thus herein, lactose is considered to be a digestible carbohydrate. However, also other digestible carbohydrates such as glucose, sucrose, fructose, galactose, maltose, starch and maltodextrin may be present. Preferably the present nutritional composition does not comprise high amounts of digestible carbohydrates other than lactose. When in liquid form, e.g. as a ready-to-feed liquid, the nutritional composition preferably comprises 6.0 to 30 g digestible carbohydrate per 100 ml, more preferably 6.0 to 20, even more preferably 7.0 to 10.0 g per 100 ml. Based on dry weight the present nutritional composition preferably comprises 40 to 80 wt.%, more preferably 40 to 65 wt.% digestible carbohydrates. Based on total calories the nutritional composition comprises 9 to 20 g digestible carbohydrates per 100 kcal, more preferably 9 to 15 g.

The present nutritional composition preferably comprises lipid. The lipid of the present nutritional composition provides 3 to 7 g per 100 kcal of the nutritional composition, preferably the lipid provides 4 to 6 g per 100 kcal. When in liquid form, e.g. as a ready-to-feed liquid, the nutritional composition preferably comprises 2.1 to 6.5 g lipid per 100 ml, more preferably 3.0 to 4.0 g per 100 ml. Based on dry weight the present nutritional composition preferably comprises 12.5 to 40 wt.% lipid, more preferably 19 to 30 wt.%. Preferably the lipid comprises the essential fatty acids alpha-linolenic acid (ALA), linoleic acid (LA) and/or long chain polyunsaturated fatty acids (LC-PUFA). The LC-PUFA, LA and/or ALA may be provided as free fatty acids, in triglyceride form, in diglyceride form, in monoglyceride form, in phospholipid form, or as a mixture of one of more of the above. Preferably the present nutritional composition contains at least one, preferably at least two lipid sources selected from the group consisting of rape seed oil (such as colza oil, low erucic acid rape seed oil and canola oil), high oleic sunflower oil, high oleic safflower oil, olive oil, marine oils, microbial oils, coconut oil, palm kernel oil and milk fat. Preferably the present nutritional composition comprises at least 0.2 wt.%, more preferably at least 0.4 wt.%, long chain poly unsaturated fatty acids based on total fatty acids, wherein the long chain poly unsaturated fatty acids are one or more selected from the group consisting of arachidonic acid, docosahexaenoic acid, eicosapentaenoic acid. Preferably the present nutritional composition comprises at most 2 wt.% long chain poly unsaturated fatty acids, more preferably at most 1 wt.%, based on total fatty acids of long chain poly unsaturated fatty acids, wherein the long chain poly unsaturated fatty acids are one or more selected from the group consisting of arachidonic acid, docosahexaenoic acid, eicosapentaenoic acid. Herein, the wt.% of long chain poly unsaturated fatty acids refers to the sum of arachidonic acid, docosahexaenoic acid and eicosapentaenoic acid.

Preferably the present nutritional composition comprises protein. The protein is preferably selected from the group consisting of non-human animal proteins, preferably milk proteins. Preferably the present nutritional composition comprises one or more selected from the group consisting of whey, whey protein, whey protein hydrolysate, casein and casein hydrolysate. The nutritional composition preferably contains casein, and/or whey protein, more preferably bovine whey proteins and/or bovine casein. The nutritional composition preferably comprises casein and whey proteins in a weight ratio casein:whey protein of 10:90 to 90:10, more preferably 20:80 to 80:20, even more preferably 35:65 to 55:45.

The nutritional composition of the present invention preferably provides protein in an amount of ding 1.25 to 4 g per 100 kcal, preferably providing 1.5 to 3 g, even more preferable 1.7 to 2.5 g per 100 kcal. When in liquid form, the nutritional composition preferably comprises 0.5 to 6.0 g, more preferably 1.0 to 3.0 g, even more preferably 1.0 to 1.5 g protein per 100 ml, most preferably 1.0 to 1.3 g protein per 100 ml. Based on dry weight the present nutritional composition preferably comprises 5 to 20 wt.% protein, preferably at least 8 wt.%, more preferably 8 to 14 wt.%, protein even more preferably 8 to 9.5 wt.% based on dry weight of the nutritional composition.

The nutritional composition of the present invention preferably provides lipid in an amount of 3 to 7 g per 100 kcal, preferably 4 to 6 g per 100 kcal, protein in an amount of 1,25 to 4 g per 100 kcal, preferably 1.5 or 1.6 to 3 g per 100 kcal, preferably 1.7 to 2.5 g per 100 kcal and digestible carbohydrate in an amount of 5 to 20 g per 100 kcal, preferably 8 to 15 g per 100 kcal of the nutritional composition. Preferably the present nutritional composition comprises lipid providing 4 to 6 g per 100 kcal, protein providing 1.6 to 1.9 g per 100 kcal, more preferably 1.75 to 1.85 g per 100 kcal and digestible carbohydrate providing 8 to 15 g per 100 kcal of the final nutritional composition.

The amount of total calories is determined by the sum of calories derived from protein, lipids, digestible carbohydrates and non-digestible oligosaccharides. Protein and carbohydrates are considered to have a caloric density of 4 kcal/g, fat of 9 kcal/g and non-digestible oligosaccharides 2 kcal/g.

The present nutritional composition is not human breast milk. The nutritional composition according to the invention or the nutritional composition used according to the invention preferably comprises other fractions, such as vitamins, minerals, trace elements and other micronutrients in order to make it a complete nutritional composition. Preferably the nutritional composition is selected from the group consisting of an infant formula, follow on formula, toddler milk or formula and growing up milk, more preferably form the group consisting of an infant formula. An infant formula is defined as a formula for use in infants and can for example be a starter formula, intended for infants of 0 to 4 to 6 months of age or a follow on formula, intended for infants of 4 to 6 months until 12 months of age. A toddler milk or growing up milk or formula is intended for children of 12 to 36 months of age. In one embodiment the nutritional composition is an infant formula. Infant formulae comprise vitamins, minerals, trace elements and other micronutrients according to international directives.

In one embodiment the nutritional composition is in a liquid form. In another embodiment the nutritional composition is a powder suitable for making a liquid nutritional composition after reconstitution with an aqueous solution, preferably with water. Preferably the nutritional composition is a powder, suitable for reconstitution with water to a liquid. Preferably the infant or toddler formula is a powder to be reconstituted with water. Preferably the liquid composition has a viscosity below 100 mPa.s, more preferably below 60 mPa.s, more preferably below 35 mPa.s, even more preferably below 6 mPa.s as measured in a Brookfield viscometer at 20°C at a shear rate of 100 s⁻¹. A low viscosity is important for infant or follow on formula, since it mimics the viscosity of breast milk and can then be administered via a teat.

In order to meet the caloric requirements of an infant or toddler, the nutritional composition preferably comprises 45 to 200 kcal/100 ml liquid. For infants the nutritional composition has more preferably 60 to 90 kcal/100 ml liquid, even more preferably 65 to 75 kcal/100 ml liquid. This caloric density ensures an optimal ratio between water and calorie consumption. For toddlers, human subjects with an age from 12 to 36 months, the nutritional composition more preferably has a caloric density from 45 to 65, even more preferably from 50 to 60 kcal/100 ml. The osmolarity of the present composition is preferably from 150 to 420 mOsmol/l, more preferably from 260 to 320 mOsmol/l. The low osmolarity aims to further reduce the gastrointestinal stress.

When the nutritional composition is in a liquid form, the preferred volume administered on a daily basis is in the range of about 80 to 2500 ml, more preferably about 200 to 1200 ml per day. Preferably, the number of feedings per day is from 1 to 10, preferably from 3 to 8. In one embodiment the nutritional composition is administered daily for a period of at least 2 days, preferably for a period of at least 4 weeks, preferably for a period of at least 8 weeks, more preferably for a period of at least 12 weeks, in a liquid form wherein the total volume administered daily is from 200 ml to 1200 ml and wherein the number of feedings per day is from 1 to 10.

The pH of the present nutritional composition is preferably from 5.0 to 7.5, more preferably from 5.0 to 6.5, most preferably from 5.5 to 6.3.

### Application

Preferably the present nutritional composition is suitable for, or suitable for administration to, a human subject. In one embodiment, the present nutritional composition is suitable for infants and/or young children. In one embodiment the present nutritional composition is for use in providing nutrition to human subjects with an age of 0 to 36 months. Young children, or toddlers, are defined as human subjects with an age of 12 to 36 months. Infants are defined as human subjects with an age of below 12 months. So in other words, the present nutritional composition is suitable for human subjects with an age of 0 to 36 months. Wherever in this description the term "infants and/or young children" is used, this can be replaced by "human subjects with an age of 0 to 36 months". Healthy full term infants are born with a supply of iron that usually lasts for 4 to 6 months. Preferably the present nutritional composition is suitable for a human subject with an age of 4 months to 36 months. In one embodiment the present nutritional composition is preferably for use in providing nutrition to a human subject with an age of 4 months to 36 months. These infants or young children have a higher need for iron and are therefore more prone to suffer from iron deficiency or anaemia.

Preterm infants have less iron stores, which are built up in the third trimester of pregnancy. Preterm infants, defined as infants born before week 37 of gestation, preferably before week 32, are in particular at risk of iron deficiency or anaemia. In a preferred embodiment, the present nutritional composition is suitable for a preterm infant, preferably for a preterm infant born before week 37 of gestation, more preferably for a preterm infant born before week 32 of gestation.

In one embodiment, the present nutritional composition is suitable for, or suitable for administration to, pregnant women. Pregnant women are in higher need for iron and are therefore more prone to suffer from iron deficiency or anaemia.

The present nutritional composition is preferably enterally administered, more preferably orally.

In one embodiment the present nutritional composition is for use in treating or preventing anaemia and/or iron deficiency.

In one embodiment the present nutritional composition is for use in increasing iron absorption, iron bioaccessibility and/or iron bioavailability, more preferably iron bioavailability.

In one embodiment the present nutritional composition is for improving cognitive development, improving motor development and/or improving socio-emotional development in a human subject with an age of 0 to 36 months or for preventing cognitive disorders, motor disorders and/or socio-emotional disorders in a human subject with an age of 0 to 36 months. In one embodiment the present nutritional composition is preferably for human subjects with an age of 0 to 36 months suffering from iron deficiency or anaemia or human subjects with an age of 0 to 36 months that are at risk of iron deficiency or anaemia. More preferably, in one embodiment the present nutritional composition is for improving cognitive development in a human subject with an age of 0 to 36 months or preventing cognitive disorders in a human subject with an age of 0 to 36 months.

Bioaccessibility is the amount of an ingested nutrient that is potentially available for absorption, and is dependent on digestion and/or release from the food matrix. Bioavailability is the amount of an ingested nutrient that is absorbed and available for physiological functions, and is dependent on digestion and/or release from the food matrix, absorption by intestinal cells and transport to the body cells. Absorption is the uptake of a nutrient into the cell, and is dependent on digestion and/or release form the food matrix.

Anaemia is a decrease in number of red blood cells or less than the normal quantity of hemoglobin in blood. In the present invention anaemia refers in particular to iron deficiency anaemia, i.e. anaemia caused by insufficient iron bioavailability. Iron-deficiency anaemia is caused by insufficient dietary intake and absorption of iron and causes approximately half of all anaemia cases in the world. According to the WHO anaemia is defined as a hemoglobin content of less than 6.83 mmol/l blood in infants or young children of 6 months to 5 years, of less than 7.13 mmol/l in children of 5 to 11 years of age, of less than 7.45 mmol/l in teens of 12 to 14 years of age, of less than 7.45 mmol/l in non-pregnant women with age above 15 years, of less than 6.83 mmol/l in pregnant women, and of less than 8.07 mmol/l in men above 15 years of age. Symptoms are pallor, fatigue, lightheadedness and weakness. Other symptoms can be headaches, trouble sleeping, loss of appetite, paleness, reduced resistance to infection, fragile nails. Iron-deficiency anaemia for infants in their earlier stages of development has greater consequences than it does for adults. An infant made severely iron-deficient during its earlier life cannot recover to normal iron levels even with iron therapy. Iron-deficiency anaemia affects neurological development by decreasing learning ability, negatively altering motor functions and negatively effecting socioemotional functioning as behavior. Additionally, iron-deficiency anaemia has a negative effect on physical growth. In pregnant women, of which it is estimated that 50% suffers from iron deficiency or anaemia, there is an increased need for iron. Anaemia may increase the risk of preterm or small birth weight babies.

Iron deficiency (sideropaenia or hypoferreamia) is a stage preceding iron deficiency anaemia. The body has less than adequate iron levels. It can for example be determined by measuring an abnormal value for at least two of the three following indicators, serum ferritin, transferrin saturation, and free erythrocyte protoporphyrin, while still having a haemoglobin content above the threshold for anaemia. Iron deficiency anaemia is abnormal values of 2 out of 3 indicators with anaemia (a haemoglobin content below the threshold for anaemia).

In the context of the present invention, 'prevention' of a disease or certain disorder also means 'reduction of the risk' of a disease or certain disorder and also means 'treatment of a human subject at risk' of said disease or said certain disorder.

### Example 1: Combination of fermented Infant Milk Formula with increased concentrations of lactose has a synergistic effect on iron bioavailability

Iron bioavailability was assessed in a validated Caco-2 cell culture model. Cells (at passages 25-50) were seeded at a density of 50,000 cell/cm² in 6 well plates. The cells were grown in Dulbecco's Modified Eagle Medium with 10% v/v Heat Inactivated Fetal Calf Serum, 0.1 mM Non Essential Amino Acids, 1mM Sodium Pyruvate and 1% antibiotic solution (penicillin/streptomycin). The cells were maintained at 37°C in an incubator with a 5% CO₂ - 95% air atmosphere and the medium was changed twice a week. The cells were used in the iron uptake experiments at 14-d post seeding. Infant formula was prepared according to commercial specifications. Cells were incubated with different infant formulas 30 x diluted in serum free cell culture medium. After 24 h cells were harvested and lysed. In brief, the 6 well plates were kept on ice during the whole procedure and the cells were washed 2x with ice cold PBS with Ca/Mg and washed once with ice cold PBS without Ca/Mg. The cells were scraped from the bottom of the plate in 0.5ml lysis buffer containing 50 mM Tris-HCl, 150mM NaCl, 0.5% Triton X-100 and protease inhibitor cocktail (Roche) at pH 7.5. The plates were placed on a rocking platform for 45 minutes to allow cell lysis. The lysed cells were resuspended by pipetting 3 x through a 1 ml pipette tip and centrifuged in an Eppendorf table centrifuge at 15,000rpm (maximum speed) for 15 minutes to remove the cytoskeleton and nuclei. The supernatants were analysed for protein concentration using the BCA protein determination method (Pierce). Additionally ferritin concentration, which is used as a parameter for iron uptake by Caco-2 cells, was determined by an enzyme linked immunosorbent assay (AssayPro). Ferritin was calculated as ng of ferritin per mg cellular protein. Ferritin concentration was normalised to a concentration of 1 mg iron/100ml.

The following infant milk formulae (IMF) were tested:
IMF1: An IMF similar to the commercially available LACTOFIDUS^{®} 1, except that no fermented milk-derived product is present. This IMF comprises per 100 ml: 3.9 g lactose and 4.1 g maltodextrin, 0.9 mg iron.
IMF2: An IMF similar to IMF1 but comprising per 100 ml 8 g lactose and no maltodextrin.
IMF3: Commercially available NUTRILON^{®} 2. This unfermented IMF comprises per 100 ml 6 g lactose, 1 mg iron and 0.8 g non-digestible oligosaccharides in the form of a mixture of galactooligosaccharides (source VIVINAL^{®} GOS) and long chain fructooligosaccharides (source RAFTILIN^{®} HP) in a 9:1 wt ratio.
IMF4: Commercially available LACTOFIDUS^{®} 1, being a 100 wt.% fermented infant formula. This IMF comprises per 100 ml 3.9 g lactose and 4.1 g maltodextrin, 0.9 mg iron. A small amount of galactooligosaccharide (about 0.11 g/100 ml) produced during fermentation is also present. The amount of the sum lactic acid and lactate (of which over 95 % is on the L form, is 0.15 g/100 ml (being about 1.1 wt.% based on dry weight). The pH is about 5.8.
IMF5: An IMF similar to IMF 4, but comprising per 100 ml 8 g lactose and no maltodextrin. This IMF is an inventive composition according to the present invention.
IMF6: An IMF according to the present invention comprising 30 wt.% fermented infant formula similar to IMF5 and the rest 70 wt.% making up a non-fermented infant formula. This IMF comprises per 100 ml 7.1 g lactose, and 0.53 mg iron and 0.83 g non-digestible oligosaccharides in the form of a mixture of galactooligosaccharides (source VIVINAL^{®} GOS) and long chain fructooligosaccharides (source RAFTILIN^{®} HP) and galactooligosaccharides derived from the fermented infant formula, and 0.045 g lactic acid and lactate (being about 0.33 wt.% based on dry weight). The pH is about 6.2.

The whey protein/casein weight ratio's of these IMFs were all in the range of 1 to 1.5.

The iron source was ferrous sulphate in all IMF. No effect on pH in the medium of the cell culture was observed.

The results are shown in Table 1. It can be deduced that in a non-fermented infant formula iron bioavailability is increased in the presence of high lactose instead of low lactose with maltodextrin (IMF 2 compared with IMF 1). The subsequent presence of non-digestible oligosaccharides reduces the iron bioavailability (IMF 3 compared with IMF 2).

A fermented infant formula improves the bioavailability of iron compared to a non-fermented infant formula (IMF 4 compared with IMF 1). Surprisingly the presence of high concentration of lactose in a fermented infant formula strongly increased the iron bioavailability to a much higher extent than based on the presence of ferment or lactose alone (IMF 5 versus IMF 2 and 4).

Unexpectedly using an IMF with also non-digestible oligosaccharides in a nutritional composition comprising both lactose and fermented milk-derived product, iron uptake was not decreased and the normalised iron uptake was increased (IMF 6 versus IMF 5 compared to IMF 3 versus IMF 2).

**Table 1: Bioavailability of iron from different infant formulas as measured by ferritin concentration in Caco-2 cells.**

| IMF | Ferment (wt. %) | Lactose 6 g/100 ml or above | Iron (mg/ 100 ml) | Non-digestible oligosaccharides (g/100ml) | Normalised iron uptake* |
|---|---|---|---|---|---|
| 1 | - | - | 0.9 | - | 56.35 |
| 2 | - | + | 0.9 | - | 70.15 |
| 3 | - | + | 1 | 0.8 | 47.57 |
| 4 | 100 | - | 0.9 | 0.11 | 73.07 |
| 5 | 100 | + | 0.9 | 0.11 | 156.7 |
| 6 | 30 | + | 0.53 | 0.83 | 254.6 |

| | | | | | |
|---|---|---|---|---|---|
| * ng ferritin/(mg protein x mg iron/100ml) | | | | | |

These results are indicative for use of a composition comprising a fermented milk-derived product, fermented by lactic acid producing bacteria, and high lactose concentration in treating or preventing anaemia and/or iron deficiency or for use in increasing iron absorption, iron bioaccessibility and/or iron bioavailability.

### Example 2:

Powdered infant formula, comprising per 100 g
- 8.9 g protein (whey protein/casein in a wt/wt ratio of about 1)
- 24.5 g fat
- 54.3 g carbohydrates, of which 51.5 g lactose
- 5.8 g non-digestible oligosaccharides, being a mix of short chain galactooligosaccharides (scGOS) and long chain fructooligosaccharides, of which about 4.1 classifies as dietary fiber, the rest being indigestible disaccharides present in the scGOS, which is classified as carbohydrates
- 3.9 mg iron (Fe²⁺; source ferrous sulphate)
- other minerals, trace elements and micronutrient according to international guidelines for infant and follow on formula.

Of this composition 30 wt.% based on dry weight is derived from the commercially available GALLIA LACTOFIDUS 1, which is a fermented formula. The final composition comprises about 0.33 g lactic acid and lactate based on dry weight, of which at least 95% is L-lactate/lactic acid.

The powder is packaged with instructions to reconstitute 3 scoops (13.7 g powder) with water up to 100 ml, yielding a formula with 66 kcal/100 ml. The pH is about 6.2.

## Claims

1. A nutritional composition comprising
a) a milk-derived product that is fermented by lactic acid producing bacteria, the fermented milk-derived product comprising lactic acid and/or lactate, and
b) at least 6 g lactose per 100 ml nutritional composition and/or at least 40 wt.% lactose based on dry weight of the nutritional composition, and
c) iron
for use in treating and/or preventing anaemia and/or treating and/or preventing iron deficiency in a human subject.

2. A nutritional composition comprising
a) a milk-derived product that is fermented by lactic acid producing bacteria, the fermented milk-derived product comprising lactic acid and/or lactate, and
b) at least 6 g lactose per 100 ml nutritional composition and/or at least 40 wt.% lactose based on dry weight of the nutritional composition, and
c) iron
for use in increasing iron absorption, increasing iron bioaccessibility and/or increasing iron bioavailability in a human subject.

3. The nutritional composition for use according to any one of claims 1-2 wherein the human subject is selected from the group consisting of human subjects with an age of 0 to 36 months and pregnant women.

4. A nutritional composition comprising
a) a milk-derived product that is fermented by lactic acid producing bacteria, the fermented milk-derived product comprising lactic acid and/or lactate, and
b) at least 6 g lactose per 100 ml nutritional composition and/or at least 40 wt.% lactose based on dry weight of the nutritional composition, and
c) iron
for use in improving cognitive development, motor development and/or socio-emotional development in a human subject with an age of 0 to 36 months or for preventing cognitive disorders, motor disorders and/or socio-emotional disorders in a human subject with an age of 0 to 36 months.

5. The nutritional composition for use according to any one of claims 1 to 4, wherein the nutritional composition comprises iron in a concentration of 0.4 to 0.7 mg per 100 ml nutritional composition and/or of 0.03 to 0.055 mg per g dry weight of the nutritional composition.

6. The nutritional composition for use according to any one of claims 1-5, wherein the nutritional composition comprises 0.1 to 1.5 wt.% of the sum of lactic acid and lactate based on dry weight of the nutritional composition, and/or 0.01 to 0.20 g of the sum of lactic acid and/or lactate per 100 ml nutritional composition, and in case the source of iron is ferrous lactate, the nutritional composition comprises from 0.1 to 1.6 wt.% lactic acid and/or lactate and/or 0.01 to 0.21 g lactic acid and/or lactate per 100 ml of nutritional composition, wherein the sum of L-lactic acid and L-lactate is more than 50 wt.% based on the sum of total lactic acid and lactate.

7. A nutritional composition comprising
a) 0.1 to 1.5 wt.% of the sum of lactic acid and lactate based on dry weight of the nutritional composition, and/or 0.01 to 0.20 g of the sum of lactic acid and/or lactate per 100 ml nutritional composition, and in case the source of iron is ferrous lactate, the nutritional composition comprises from 0.1 to 1.6 wt.% lactic acid and/or lactate and/or 0.01 to 0.21 g lactic acid and/or lactate per 100 ml of nutritional composition, wherein the sum of L-lactic acid and L-lactate is more than 50 wt.% based on the sum of total lactic acid and lactate, and
b) at least 6 g lactose per 100 ml nutritional composition and/or at least 40 wt.% lactose based on dry weight of nutritional composition and
c) iron in a concentration of 0.4 to 0.7 mg per 100 ml nutritional composition and/or of 0.03 to 0.055 mg per g dry weight of nutritional composition.

8. The nutritional composition for use according to any one of claims 1-6 or the nutritional composition according to claim 7, wherein the nutritional composition comprises at least 25 wt.% based on dry weight of the nutritional composition of a milk-derived product that is fermented by lactic acid producing bacteria comprising lactic acid and/or lactate.

9. The nutritional composition for use according to any one of claims 1-6 and 8 or the nutritional composition according to claim 7 or 8, wherein the fermented milk-derived nutritional composition further comprises at least one selected from the group consisting of whey, whey protein, whey protein hydrolysate, casein and casein hydrolysate.

10. The nutritional composition for use according to any one of claims 1-6 and 8-9 or the nutritional composition according to any one of claims 7-9, wherein the nutritional composition comprises *Streptococcus thermophilus* and/or *Bifidobacterium breve.*

11. The nutritional composition for use according to any one of claims 1-6 and 8-10 or the nutritional composition according to any one of claims 7-10, wherein the lactic acid producing bacteria in the nutritional composition are inactivated and/or non-replicating.

12. The nutritional composition for use according to any one of claims 1-6 and 8-11 or the nutritional composition according to any one of claims 7-11, wherein the nutritional composition comprises an iron source selected from the group consisting of ferrous sulphate, ferrous lactate, ferrous gluconate, ferrous bisglycinate, ferrous citrate, ferrous fumarate, ferric diphosphate, and ferric ammonium citrate.

13. The nutritional composition for use according to any one of claims 1-6 and 8-12 or the nutritional composition according to any one of claims 7-12, wherein the nutritional composition comprises at least one, preferably at least two, non-digestible oligosaccharides selected from the group consisting of galactooligosaccharides, fructooligosaccharides, uronic acid oligosaccharides, glucooligosaccharides, xylooligosaccharides, mannanoligosaccharides, arabino-oligosaccharides, glucomannooligosaccharides, galactomannooligosaccharides, soy oligosaccharides, isomaltooligosaccharides, non-digestible dextrin, arabinogalactooligosaccharides, gentiooligosaccharides, nigerooligosaccharides, chitooligosaccharides, sialyloligosaccharides, and fucooligosaccharides.

14. The nutritional composition for use according claim 13 or the nutritional composition according to claim 13, wherein the at least one non-digestible oligosaccharide is selected from the group consisting of galactooligosaccharides and fructooligosaccharides.

15. The nutritional composition for use according to any one of claims 1-6 and 8-14 or the nutritional composition according to any one of claims 7-14, wherein the nutritional composition comprises 5 to 20 wt.% protein, based on dry weight of the nutritional composition.

16. The nutritional composition for use according to any one of claims 1-6 and 8-15 or the nutritional composition according to any one of claims 8-15, wherein the nutritional composition comprises protein, lipid and digestible carbohydrates, and the protein provides 1.25 to 4 g per 100 kcal of the nutritional composition, the lipid provides 3 to 7 g per 100 kcal of the nutritional composition and the digestible carbohydrate provides 5 to 20 g per 100 kcal of the nutritional composition.

17. The nutritional composition for use according to claim 16 or the nutritional composition according to claim 16, wherein the lipid comprises at least 0.2 wt.% long chain poly unsaturated fatty acids based on total fatty acids, wherein the long chain poly unsaturated fatty acids are one or more selected from the group consisting of arachidonic acid, docosahexaenoic acid, eicosapentaenoic acid.

18. The nutritional composition for use according to any one of claims 1-6 and 8-17 or the nutritional composition according to any one of claims 7-17, for use in providing nutrition to human subjects with an age of 0 to 36 months or pregnant women.

19. The nutritional composition for use according to any one of claims 1-6 and 8-18 or the nutritional composition according to any one of claims 7-18, wherein the nutritional composition is selected from the group consisting of infant formula, follow on formula and growing up milk.

20. The nutritional composition for use according to any one of claims 1-6 and 8-19 or the nutritional composition according to any one of claims 7-19, wherein the nutritional composition is a powder, suitable for reconstitution with water to a liquid.

## Patentansprüche

1. Nährstoffzusammensetzung umfassend:
a) ein von Milch herrührendes Produkt, das durch Milchsäure produzierende Bakterien fermentiert ist, wobei das fermentierte von Milch herrührende Produkt Milchsäure und/oder Laktat umfasst, und
b) wenigstens 6 g Laktose pro 100 ml Nährstoffzusammensetzung und/oder wenigstens 40 Gew.-% Laktose basierend auf einem Trockengewicht der Nährstoffzusammensetzung, und
c) Eisen
zur Verwendung in der Behandlung und/oder Vermeidung von Blutarmut und/oder Behandlung und/oder Vermeidung von Eisenmangel bei einem Menschen.

2. Nährstoffzusammensetzung, umfassend:
a) ein von Milch herrührendes Produkt, das durch Milchsäure produzierende Bakterien fermentiert ist, wobei das fermentierte von Milch herrührende Produkt Milchsäure und/oder Laktat umfasst, und
b) wenigstens 6 g Laktose pro 100 ml Nährstoffzusammensetzung und/oder wenigstens 40 Gew.-% Laktose basierend auf einem Trockengewicht der Nährstoffzusammensetzung, und
c) Eisen
zur Verwendung bei der Erhöhung einer Eisenabsorption, Erhöhung einer Eisenbiozugänglichkeit und/oder Erhöhung einer Eisenbioverfügbarkeit bei einem Menschen.

3. Nährstoffzusammensetzung zur Verwendung nach einem der Ansprüche 1 bis 2, wobei der Mensch ausgewählt ist aus der Gruppe bestehend aus Menschen mit einem Alter von 0 bis 36 Monaten und schwangeren Frauen.

4. Nährstoffzusammensetzung, umfassend:
a) ein von Milch herrührendes Produkt, das durch Milchsäure produzierende Bakterien fermentiert ist, wobei das fermentierte von Milch herrührende Produkt Milchsäure und/oder Laktat umfasst, und
b) wenigstens 6 g Laktose pro 100 ml Nährstoffzusammensetzung und/oder wenigstens 40 Gew.-% Laktose basierend auf einem Trockengewicht der Nährstoffzusammensetzung, und
c) Eisen
zur Verwendung bei einer Verbesserung einer kognitiven Entwicklung, motorischen Entwicklung und/oder sozio-emotionalen Entwicklung bei einem Menschen mit einem Alter von 0 bis 36 Monaten oder zur Verhinderung von kognitiven Störungen, motorischen Störungen und/oder sozio-emotionalen Störungen bei einem Menschen mit einem Alter von 0 bis 36 Monaten.

5. Nährstoffzusammensetzung zur Verwendung nach einem der Ansprüche 1 bis 4, wobei die Nährstoffzusammensetzung Eisen in einer Konzentration von 0,4 bis 0,7 mg pro 100 ml Nährstoffzusammensetzung und/oder 0,03 bis 0,055 mg pro g Trockengewicht der Nährstoffzusammensetzung umfasst.

6. Nährstoffzusammensetzung zur Verwendung nach einem der Ansprüche 1 bis 5, wobei die Nährstoffzusammensetzung 0,1 bis 1,5 Gew.-% der Summe aus Milchsäure und Laktat, basierend auf einem Trockengewicht der Nährstoffzusammensetzung, und/oder 0,01 bis 0,20 g der Summe aus Milchsäure und/oder Laktat pro 100 ml Nährstoffzusammensetzung umfasst, und im Falle, dass die Eisenquelle Eisen(II)laktat ist, die Nährstoffzusammensetzung 0,1 bis 1,6 Gew.-% Milchsäure und/oder Laktat und/oder 0,01 bis 0,21 Milchsäure und/oder Laktat pro 100 ml Nährstoffzusammensetzung umfasst, wobei die Summe aus L-Milchsäure und L-Laktat mehr als 50 Gew.-%, basierend auf der Summe aus gesamter Milchsäure und Laktat, ist.

7. Nährstoffzusammensetzung, umfassend:
a) 0,1 bis 1,5 Gew.-% der Summe aus Milchsäure und Laktat, basierend auf einem Trockengewicht der Nährstoffzusammensetzung, und/oder 0,01 bis 0,20 g der Summe aus Milchsäure und/oder Laktat pro 100 ml Nährstoffzusammensetzung, und im Falle, wo die Eisenquelle Eisen(II)laktat ist, die Nährstoffzusammensetzung 0,1 bis 1,6 Gew.-% Milchsäure und/oder Laktat und/oder 0,01 bis 0,21 Milchsäure und/oder Laktat pro 100 ml Nährstoffzusammensetzung umfasst, wobei die Summe aus L-Milchsäure und L-Laktat mehr als 50 Gew.-%, basierend auf der Summe der gesamten Milchsäure und des gesamten Laktats, ist, und
b) wenigstens 6 g Laktose pro 100 ml Nährstoffzusammensetzung und/oder wenigstens 40 Gew.-% Laktose basierend auf einem Trockengewicht der Nährstoffzusammensetzung, und
c) Eisen in einer Konzentration von 0,4 bis 0,7 mg pro 100 ml Nährstoffzusammensetzung und/oder 0,03 bis 0,055 mg pro g Trockengewicht der Nährstoffzusammensetzung.

8. Nährstoffzusammensetzung zur Verwendung nach einem der Ansprüche 1 bis 6 oder der Nährstoffzusammensetzung nach Anspruch 7, wobei die Nährstoffzusammensetzung wenigstens 25 Gew.-%, basierend auf einem Trockengewicht der Nährstoffzusammensetzung, eines von Milch herrührenden Produkts umfasst, das durch Milchsäure produzierende Bakterien fermentiert ist, umfassend Milchsäure und/oder Laktat.

9. Nährstoffzusammensetzung zur Verwendung nach einem der Ansprüche 1 bis 6 und 8 oder die Nährstoffzusammensetzung nach Anspruch 7 oder 8, wobei die fermentierte von Milch herrührende Nährstoffzusammensetzung ferner wenigstens eines umfasst, das ausgewählt ist aus der Gruppe bestehend aus Molke, Molkeprotein, Molkeproteinhydrolysat, Casein und Caseinhydrolysat.

10. Nährstoffzusammensetzung zur Verwendung nach einem der Ansprüche 1 bis 6 und 8 bis 9 oder die Nährstoffzusammensetzung nach einem der Ansprüche 7-9, wobei Nährstoffzusammensetzung *Streptococcus thermophilus* und/oder *Bifidobacterium breve* umfasst.

11. Nährstoffzusammensetzung zur Verwendung nach einem der Ansprüche 1-6 und 8-10 oder die Nährstoffzusammensetzung nach einem der Ansprüche 7-10, wobei die Milchsäure produzierenden Bakterien in der Nährstoffzusammensetzung inaktiviert und/oder nicht-replizierend sind.

12. Nährstoffzusammensetzung zur Verwendung nach einem der Ansprüche 1-6 und 8-11 oder die Nährstoffzusammensetzung nach einem der Ansprüche 7-11, wobei die Nährstoffzusammensetzung eine Eisenquelle umfasst, die ausgewählt ist aus der Gruppe bestehend aus Eisen(II)sulfat, Eisen(II)laktat, Eisen(II)glukonat, Eisen(II)bisglycinat, Eisen(II)citrat, Eisen(II)fumarat, Eisen(III)diphosphat und Eisen(III)ammoniumcitrat.

13. Nährstoffzusammensetzung zur Verwendung nach einem der Ansprüche 1-6 und 8-12 oder die Nährstoffzusammensetzung nach einem der Ansprüche 7-12, wobei die Nährstoffzusammensetzung wenigstens ein, bevorzugt wenigstens zwei, nicht-verdauliches Oligosaccharid umfasst, ausgewählt aus der Gruppe bestehend aus Galaktooligosacchariden, Fruktooligosacchariden, Uronsäureoligosacchariden, Glukooligosacchariden, Xylooligosacchariden, Mannanoligosacchariden, Arabino-Oligosacchariden, Glukomannooligosacchariden, Galaktomannooligosacchariden, Sojaoligosacchariden, Isomaltooligosacchariden, nicht-verdaulichem Dextrin, Arabinogalactooligosacchariden, Gentiooligosacchariden, Nigerooligosacchariden, Chitooligosacchariden, Sialyloligosacchariden und Fucoolisosacchariden.

14. Nährstoffzusammensetzung zur Verwendung nach Anspruch 13 oder die Nährstoffzusammensetzung nach Anspruch 13, wobei das wenigstens eine nicht-verdauliche Oligosaccharid ausgewählt ist aus der Gruppe bestehend aus Galaktooligosacchariden und Fruktooligosacchariden.

15. Nährstoffzusammensetzung zur Verwendung nach einem der Ansprüche 1-6 und 8-14 oder die Nährstoffzusammensetzung nach einem der Ansprüche 7-14, wobei die Nährstoffzusammensetzung 5 bis 20 Gew.-% Protein, basierend auf einem Trockengewicht der Nährstoffzusammensetzung, umfasst.

16. Nährstoffzusammensetzung zur Verwendung nach einem der Ansprüche 1-6 und 8-15 oder die Nährstoffzusammensetzung nach einem der Ansprüche 8-15, wobei die Nährstoffzusammensetzung Protein, Lipid und verdauliche Kohlenhydrate umfasst, und wobei das Protein 1,25 bis 4 g pro 100 kcal der Nährstoffzusammensetzung liefert, das Lipid 3 bis 7 g pro 100 kcal der Nährstoffzusammensetzung liefert und das verdauliche Kohlenhydrat 5-20 g pro 100 kcal der Nährstoffzusammensetzung liefert.

17. Nährstoffzusammensetzung zur Verwendung nach Anspruch 16 oder die Nährstoffzusammensetzung nach Anspruch 16, wobei das Lipid wenigstens 0,2 Gew.-% langkettige polyungesättigte Fettsäuren, basierend auf gesamten Fettsäuren, umfasst, wobei die langkettigen polyungesättigten Fettsäuren eine oder mehrere sind, die ausgewählt sind aus der Gruppe, bestehend aus Arachidonsäure, Docosahexaensäure, Eicosapentaensäure.

18. Nährstoffzusammensetzung zur Verwendung nach einem der Ansprüche 1-6 und 8-17 oder die Nährstoffzusammensetzung nach einem der Ansprüche 7-17, zur Verwendung bei der Ernährung von Menschen mit einem Alter von 0 bis 36 Monaten oder schwangeren Frauen.

19. Nährstoffzusammensetzung zur Verwendung nach einem der Ansprüche 1-6 und 8-18 oder die Nährstoffzusammensetzung nach einem der Ansprüche 7-18, wobei die Nährstoffzusammensetzung ausgewählt ist aus der Gruppe bestehend aus Kleinkindmilchnahrung, Folgemilchnahrung und Wachstumsmilch.

20. Nährstoffzusammensetzung zur Verwendung nach einem Ansprüche 1-6 und 8-19 oder die Nährstoffzusammensetzung nach einem der Ansprüche 7-19, wobei die Nährstoffzusammensetzung ein Pulver ist, geeignet zur Rekonstitution mit Wasser zu einer Flüssigkeit.

## Revendications

1. Composition nutritionnelle comprenant
a) un produit dérivé du lait qui est fermenté par des bactéries produisant de l'acide lactique, le produit dérivé du lait fermenté comprenant de l'acide lactique et/ou du lactate, et
b) au moins 6 g de lactose pour 100 ml de composition nutritionnelle et/ou au moins 40% en poids de lactose sur la base du poids sec de la composition nutritionnelle, et
c) du fer
à utiliser pour traiter et/ou prévenir une anémie et/ou traiter et/ou prévenir une déficience en fer chez un sujet humain.

2. Composition nutritionnelle comprenant
a) un produit dérivé du lait qui est fermenté par des bactéries produisant de l'acide lactique, le produit dérivé du lait fermenté comprenant de l'acide lactique et/ou du lactate, et
b) au moins 6 g de lactose pour 100 ml de composition nutritionnelle et/ou au moins 40% en poids de lactose sur la base du poids sec de la composition nutritionnelle, et
c) du fer
à utiliser pour une absorption de fer croissante, une bioaccessibilité de fer croissante et/ou une biodisponibilité de fer croissante chez un sujet humain.

3. Composition nutritionnelle à utiliser selon l'une quelconque des revendications 1-2, dans lequel le sujet humain est sélectionné dans le groupe constitué de sujets humains âgés de 0 à 36 mois et de femmes enceintes.

4. Composition nutritionnelle comprenant
a) un produit dérivé du lait qui est fermenté par des bactéries produisant de l'acide lactique, le produit dérivé du lait fermenté comprenant de l'acide lactique et/ou du lactate, et
b) au moins 6 g de lactose pour 100 ml de composition nutritionnelle et/ou au moins 40% en poids de lactose sur la base du poids sec de la composition nutritionnelle, et
c) du fer
à utiliser pour améliorer le développement cognitif, le développement moteur et/ou le développement socio-émotionnel chez un sujet humain âgé de 0 à 36 mois ou pour prévenir des désordres cognitifs, des désordres moteurs et/ou des désordres socio-émotionnels chez un sujet humain âgé de 0 à 36 mois.

5. Composition nutritionnelle à utiliser selon l'une quelconque des revendications 1 à 4, dans laquelle la composition nutritionnelle comprend du fer en une concentration de 0,4 à 0,7 mg pour 100 ml de composition nutritionnelle et/ou de 0,03 à 0,055 mg par g de poids sec de la composition nutritionnelle.

6. Composition nutritionnelle à utiliser selon l'une quelconque des revendications 1-5, dans laquelle la composition nutritionnelle comprend de 0,1 à 1,5 % en poids de la somme de l'acide lactique et du lactate sur la base du poids sec de la composition nutritionnelle, et/ou de 0,01 à 0,20 g de la somme de l'acide lactique et/ou du lactate pour 100 ml de composition nutritionnelle, et dans le cas où la source de fer est du lactate ferreux, la composition nutritionnelle comprend de 0,1 à 1,6 % en poids d'acide lactique et/ou de lactate et/ou de 0,01 à 0,21 g d'acide lactique et/ou de lactate pour 100 ml de composition nutritionnelle, dans laquelle la somme d'acide L-lactique et de L-lactate est supérieure à 50 % en poids sur la base de la somme totale de l'acide lactique et du lactate.

7. Composition nutritionnelle comprenant
a) de 0,1 à 1,5 % en poids de la somme de l'acide lactique et du lactate sur la base du poids sec de la composition nutritionnelle et/ou de 0,01 à 0,20 g de la somme de l'acide lactique et/ou du lactate pour 100 ml de composition nutritionnelle, et dans le cas où la source de fer est du lactate ferreux, la composition nutritionnelle comprend de 0,1 à 1,6 % en poids d'acide lactique et/ou de lactate et/ou de 0,01 à 0,21 g d'acide lactique et/ou de lactate pour 100 ml de composition nutritionnelle, dans laquelle la somme d'acide L-lactique et de L-lactate est supérieure à 50 % en poids sur la base de la somme totale de l'acide lactique et du lactate, et
b) au moins 6 g de lactose pour 100 ml de composition nutritionnelle et/ou au moins 40 % en poids de lactose sur la base du poids sec de composition nutritionnelle, et
c) du fer en une concentration de 0,4 à 0,7 mg pour 100 ml de composition nutritionnelle et/ou de 0,03 à 0,055 mg par g de poids sec de composition nutritionnelle.

8. Composition nutritionnelle à utiliser selon l'une quelconque des revendications 1-6 ou composition nutritionnelle selon la revendication 7, dans laquelle la composition comprend au moins 25 % en poids sur la base du poids sec de la composition nutritionnelle d'un produit dérivé du lait qui est fermenté par des bactéries produisant de l'acide lactique, comprenant de l'acide lactique et/ou du lactate.

9. Composition nutritionnelle à utiliser selon l'une quelconque des revendications 1-6 et 8 ou composition nutritionnelle selon la revendication 7 ou 8, dans laquelle la composition nutritionnelle dérivée du lait fermentée comprend en outre au moins un élément sélectionné dans le groupe composé de lactosérum, protéines de lactosérum, hydrolysat de protéines de lactosérum, caséine et hydrolysat de caséine.

10. Composition nutritionnelle à utiliser selon l'une quelconque des revendications 1-6 et 8-9 ou composition nutritionnelle selon l'une quelconque des revendications 7-9, dans laquelle la composition nutritionnelle comprend *Streptococcus thermophilus* et/ou *Bifidobacterium breve.*

11. Composition nutritionnelle à utiliser selon l'une quelconque des revendications 1-6 et 8-10 ou composition nutritionnelle selon l'une quelconque des revendications 7-10, dans laquelle les bactéries produisant de l'acide lactique dans la composition nutritionnelle sont inactivées et/ou non répliquantes.

12. Composition nutritionnelle à utiliser selon l'une quelconque des revendications 1-6 et 8-11, ou composition nutritionnelle selon l'une quelconque des revendications 7-11, dans laquelle la composition nutritionnelle comprend une source de fer choisie dans le groupe comprenant sulfate ferreux, lactate ferreux, gluconate ferreux, bisglycinate ferreux, citrate ferreux, fumarate ferreux, diphosphate ferrique et citrate d'ammonium ferrique.

13. Composition nutritionnelle à utiliser selon l'une quelconque des revendications 1-6 et 8-12 ou composition nutritionnelle selon l'une quelconque des revendications 7-12, dans laquelle la composition nutritionnelle comprend au moins un, de préférence au moins deux, oligosaccharides non digestibles choisis parmi les groupes comprenant galactooligosaccharides, fructooligosaccharides, oligosaccharides d'acide uroniques, glucooligosaccharides, xylooligosaccharides, mannanoligosaccharides, arabino-oligosaccharides, glucomannooligosaccharides, galactomannooligosaccharides, oligosaccharides de soja, isomaltooligosaccharides, dextrine non digestible, arabinogalactooligosaccharides, gentiooligosaccharides, nigerooligosaccharides, chitooligosaccharides, sialyloligosaccharides et fucooligosaccharides.

14. Composition nutritionnelle à utiliser selon la revendication 13 ou composition nutritionnelle selon la revendication 13, dans laquelle le au moins un oligosaccharide non digestible est choisi dans le groupe comprenant galactooligosaccharides et fructooligosaccharides.

15. Composition nutritionnelle à utiliser selon l'une quelconque des revendications 1-6 et 8-14, ou composition nutritionnelle selon l'une quelconque des revendications 7-14, dans laquelle la composition nutritionnelle comprend de 5 à 20 % en poids de protéines, sur la base du poids sec de la composition nutritionnelle.

16. Composition nutritionnelle à utiliser selon l'une quelconque des revendications 1-6 et 8-15, ou composition nutritionnelle selon l'une quelconque des revendications 8-15, dans laquelle la composition nutritionnelle comprend des protéines, des lipides et des glucides digestibles, et les protéines représentent de 1,25 à 4 g pour 100 kcal de la composition nutritionnelle, les lipides représentent de 3 à 7 g pour 100 kcal de la composition nutritionnelle et les glucides digestibles représentent de 5 à 20 g pour 100 kcal de la composition nutritionnelle.

17. Composition nutritionnelle à utiliser selon la revendication 16 ou composition nutritionnelle selon la revendication 16, dans laquelle les lipides comprennent au moins 0,2 % en poids d'acides gras poly-insaturés à longue chaîne sur la base des acides gras totaux, dans laquelle les acides gras poly-insaturés à longue chaîne sont un ou plusieurs choisis dans le groupe comprenant acide arachidonique, acide docosahexanoïque et acide eicosapentaénoïque.

18. Composition nutritionnelle à utiliser selon l'une quelconque des revendications 1-6 et 8-17 ou composition nutritionnelle selon l'une quelconque des revendications 7-17, à utiliser pour fournir une nutrition à des sujets humains âgés de 0 à 36 mois ou à des femmes enceintes.

19. Composition nutritionnelle à utiliser selon l'une quelconque des revendications 1-6 et 8-18 ou composition nutritionnelle selon l'une quelconque des revendications 7-18, dans lequel la composition nutritionnelle est choisie dans le groupe constitué par des préparations pour nourrissons, des préparations de suite et du lait de croissance.

20. Composition nutritionnelle à utiliser selon l'une quelconque des revendications 1-6 et 8-19 ou composition nutritionnelle selon l'une quelconque des revendications 7-19, dans laquelle la composition nutritionnelle est une poudre, adaptée pour une reconstitution à l'état liquide avec de l'eau.
